# EUROPEAN PATENT APPLICATION

(11) **EP 3 381 353 A1**
(43) Date of publication of application: **03.10.2018**
(21) Application number: 17163915.6
(22) Date of filing: 30.03.2017
(51) Int. Cl.: A61B 5/00, G09B 9/00, A61B 6/00, A61B 5/055, G01R 33/54, G06F 19/00

(54) **METHOD FOR PLANNING AN IMAGING SCAN PROTOCOL**

(71) Applicant: Koninklijke Philips N.V., 5656 AE Eindhoven (NL)
(72) Inventor: UHLEMANN, Falk, 5656 AE Eindhoven (NL)
(74) Representative: de Haan, Poul Erik

(57) **Abstract**

The present disclosure relates to a method for planning an imaging scan protocol to be performed by a scanning imaging system (100), the method comprising simulating for a patient (118) by a virtual reality system (200) an imaging scan, the imaging scan following a chosen imaging scan protocol (141), recording physiological data (152) of the patient (118) during the simulating and modifying the chosen imaging scan protocol (141) based on the physiological data (152).

## Description

### FIELD OF THE INVENTION

The invention relates to a method for planning an imaging scan protocol, a computer program product and a system for planning an imaging scan protocol.

### BACKGROUND OF THE INVENTION

Hospitals, doctors, nurses and assistants do have a very limited amount of time to address concerns or questions of patients in preparation of medical procedures, like e.g. MR imaging or CT imaging. Large amount of information is available via brochures or the internet. Nevertheless imaging or therapeutic procedures are an uncomfortable situation for many patients, often simply because the patients do not know what to really expect and/or how to act.

US2011258570 A1 relates to a method for using an electronic device having a display to prepare a patient for a medical treatment, the method including the steps of: representing a world on the display, wherein one or more medical objects related to the medical treatment are placed in the world; receiving an input from the patient to select the medical objects; selecting the medical objects; and providing feedback to the patient to indicate that the medical objects have been selected.

### SUMMARY OF THE INVENTION

Various embodiments provide for a method for planning an imaging scan protocol, a computer program product and a system for planning an imaging scan protocol, as described by the subject matter of the independent claims. Advantageous embodiments are described in the dependent claims.

In one aspect, the invention relates to method for planning an imaging scan protocol to be performed by a scanning imaging system, the method comprising: simulating for a patient by a virtual reality system an imaging scan, the imaging scan following a chosen imaging scan protocol; recording physiological data of the patient during the simulating; modifying the chosen imaging scan protocol based on the physiological data.

The term "imaging scan protocol" refers to a set of computer executable instructions that can be provided to the scanning imaging system in order to control the scanning imaging system to perform an imaging operation in a desired manner. In case of magnetic resonance imaging (MRI) this encompasses one or multiple pulse sequences in a given order, wherein the for each pulse sequence control parameters for the imaging system may be given in terms of flip angles, repetition times, scan plane selections, gradient coil control commands etc. Examples of pulse sequences are a fast-field echo sequence, turbo-field echo sequence, gradient-echo sequence, inversion Echo Single-Shot EPI Gradient Echo etc.

The term "imaging scan protocol" may include anyone of: one or more of the imaging scans, one or more prescans, loading of scan protocols, performing a predetermined processing on the received MRI signals, and storing the processed MRI signals. For example, a scan protocol library maybe provided which includes a set of different imaging scan protocols. The chosen imaging scan protocol may be selected from the library and based on the recorded physiological data the chosen protocol may be automatically modified and applied on an imaging system for performing the imaging. The imaging scan protocol may also comprise instructions regarding the reconstruction of the magnetic resonance (MR) image data acquired using the imaging.

The term "imaging scan" includes both scans including only a single 2D image frame acquisition pass as well as 3D scanning techniques wherein each individual scan is performed as a time series of individual acquisition passes which are equal in terms of parameters and contrasts. For example, in case the scanning imaging system is a magnetic resonance (MR) imaging system, the term "scan" may refer to a data acquisition sequence including applying a static magnetic field and a gradient magnetic field, transmitting an RF pulse, receiving an MRI signal, storing the received MRI signal.

The term "virtual reality system" refers to a system which that used software to generate realistic images and sounds that replicates a real environment and simulate a user's physical presence in this environment. The environment is the scanner to be used for performing the imaging scan, for example a magnetic resonance imaging scanner. The simulation includes for example the movement of the patient lying on a table into the bore of the magnet of the MR scanner, as well as the situation when a region of interest of the patient is located within the bore of the scanner and is subject to an imaging scan.

Even though only pulse sequences for MRI have been mentioned, it will be understood that the general principles discussed in this disclosures generally apply to other imaging techniques like for example computer tomography, projectional radiography, diagnostic sonography, scintigraphy, positron emission tomography etc.

Embodiments of the invention may have the advantage that the total quality of the imaging is increased. The physiological data recorded during the simulating permits to the deduce information like for example the stress level the patient experiences during the simulated imaging scan. In case of a high stress level the patient may start making undesired movements and breathe faster, which may have a negative effect on the quality of the MR images acquired during a real MR scan. The straightforward compensation of low image quality would be the repetition of performing a certain part of the imaging scan or even the whole imaging scan, which would in turn stress the patient even more and additionally make the imaging system unavailable for imaging of other patients. In contrast, by means of the described method for planning the imaging scan protocol individual physiological reactions of the patient with respect to the simulated imaging scan can be considered for accordingly modifying the chosen imaging scan protocol. In turn, the total quality of the imaging may be significantly increased.

In accordance with an embodiment of the invention, the virtual reality system comprises sensors, the recording of the physiological data being performed using the sensors. This may have the advantage that the recording of the physiological data can be performed in an easy manner. Preferably no separate external devices are required here. In the preferred embodiment, the patient may even himself or herself perform the simulation and the recording of the physiological data using a virtual reality device provided to him or her. For example, the virtual reality system is comprising a virtual reality headset for performing the simulation, wherein the sensors are comprised in the headset. The sensors may be integrated in the head set.

For example, the headset may comprise a sensor which records the actual heart rate of the patient and the breathing rate of the patient. The skin conductance of the patient may be easily measured using conductivity sensors that are integrated into the headset.

In accordance with an embodiment of the invention, the method is further comprising generating a 3D model of the scanner to be used for performing the imaging scan and generating an acoustic model of the noise emissions of the scanner resulting from performing the imaging scan, the simulating comprising an optical visualization of the imaging scan using the 3D model and an acoustic simulation of the imaging scan using the acoustic model, the optical visualization and the acoustic simulation mimicking the visual and acoustic impressions on the patient when the patient is subject to the imaging scan in reality.

This may have the benefit that the patient is experiencing in a visual and acoustic manner the situation he or she will experience in reality when being subject to the imaging scan. Thus, the patient knows exactly what to expect and what to do during the imaging scan. For example, in case the patient has to perform some sort of breath holding technique during at the imaging scan, since he is trained accordingly by the simulation the patient knows in advance how to react to certain spoken breath holding commands that will be given to him by the operator or the imaging system itself during the real imaging scan. It has to be noted here that the provision of spoken commands to a patient can be automatically performed by the scanning imaging system. The provision of the commands via a loudspeaker or headphone the patient is wearing during the imaging may be part of the scanning imaging protocol. Again, the risk of scan delays due to wrong or the late response of the patient to certain spoken commands is minimized which in the turn increases the scanning imaging system availability to other patients.

The noise emissions of the scanner may be due to operations of gradient coils, pump systems used for cooling a magnet of for example the MRI system, as well as commands provided to the patient in verbally manner during the imaging scan. The optical visualization of the imaging scan using the 3-D model comprises for example the movement of the patient lying on the movable bed of the scanner into the bore of the scanner.

In accordance with an embodiment of the invention, the physiological data is comprising anyone of the following: blood pressure, skin conductance, heart rate, breath rate, breathing pattern, blood oxygen saturation, pupil width, pupil movement, muscle tension (EMG), neuronal activity (EEG), skin temperature, reaction time for performing a physical or mental activity, breath hold duration.

In accordance with an embodiment of the invention, the method is further comprising anyone of
in case a combination of values of the physiological data is above a predefined threshold level, modifying the chosen imaging scan protocol such that the combination of values of the physiological data to be expected when performing the imaging scan protocol in reality is reduced below the threshold level,
in case the combination of values of the physiological data is below a predefined threshold level, modifying the chosen imaging scan protocol such that the combination of values of the physiological data level to be expected when performing the imaging scan protocol in reality is at the threshold level.

Combinations of values of the physiological data may result in a stress level, reaction speed, and movement behavior (e.g. trembling of the patient). For example, by combining absolute values and changes of heart rate and skin conductance, the stress level of the patient can be calculated. In case the stress level is higher than the predefined threshold level, the chosen imaging protocol may be modified in such a manner that the stress level is reduced. For example, the noise emission of the gradient coils may be reduced by reducing the magnetic field generated by the gradient coils. With less noise it may be expected that the stress level of the patient may be reduced. Of course, the reduction of the magnetic field generated by the gradient coils has to be compensated by longer scan sequences or compromised image quality/contrasts such that the method has to evaluate the different impacts of scan duration on noise emission onto the patient and find an optimal combination which provides high quality image data with minimized patient stress.

Vice versa, in case the combination of certain values of the physiological data is below a predefined threshold level, the chosen imaging scan protocol can be modified in such a manner that the combination of values of the physiological data is increasing but still below the threshold level. In the above example of stress level, in case it turns out that the patient very well accommodates with the chosen imaging scan protocol, certain parameters of the imaging scan protocol may be modified to increase breath hold durations or noise emissions due to higher gradient coil magnetic fields. This in turn leads either to a reduced total imaging time or to higher quality of the acquired MR images.

In accordance with an embodiment of the invention, the modification of the chosen imaging scan protocol is comprising anyone of: a modification of the time duration of the chosen imaging scan protocol, a modification of the noise level resulting from performing the chosen imaging scan protocol, a modification of the image acquisition parameters of the chosen imaging scan protocol, a modification of the image reconstruction parameters of the chosen imaging scan protocol, a splitting of the chosen imaging scan protocol into a set of scan sequences separated in time from each other, rearranging the order of a set of scan sequences comprised in the chosen imaging scan protocol.

The rearranging of the order of the set of scan sequences may for example be performed in accordance with priorities assigned to the individual scan sequences. For example, in order to permit a certain medical diagnosis from acquired MR images, some of the scan sequences may be considered as being essential, while others scan sequences may be considered as being optional. In case that from the physiological data it can be determined that there is the risk that the patient will not be able to sit out the whole imaging process, the scan sequences may be reordered in such a manner that the most relevant scan sequences are performed first, followed by the optional and thus less relevant scan sequences.

In a similar situation when again there is the risk that a patient will not be able to sit out the whole imaging process as planned, the time duration of the chosen imaging scan protocol maybe reduced regarding some of the less relevant scan sequences. Thus, high quality image data is still obtained from the imaging sequences that are acquired in an unmodified and normal manner. For the less relevant scan sequences, image data is obtained, however with less quality.

The chosen imaging scan protocol may also be modified regarding the way images are reconstructed from the image data acquired using the scan sequences. In an example, the breathing pattern of the patient may be determined as the physiological data during the simulation. Using the breathing pattern, respective motion compensation can be performed on the image data acquired during the real imaging scan.

In accordance with an embodiment of the invention, the method further comprises analyzing the breathing pattern for identifying the time and/or amplitude behavior of reoccurring breathing induced movement states of the patient's body, wherein the modifying of the chosen imaging scan protocol is performed such that image data acquisition is adapted to and/or synchronized with the time behavior.

The imaging scan protocol may for example be adapted to the pre-recorded patient specific pattern in such a manner that images are always taken during the same phase of breathing. As a consequence, all recorded images show the same geometric conditions of the body of the patient which simplifies a high quality image reconstruction process.

In accordance with an embodiment of the invention, the method further comprises performing the imaging scan using the imaging scan protocol, the performing of the imaging scan resulting in image data, the method further comprising performing a motion compensation of the image data using the breathing pattern. In this embodiment, even though images may not always be taken during the same phase of breathing, the knowledge of the breathing pattern permits a more robust tracking of motion and motion compensation when reconstructing the acquired images. This even permits to support a motion model building (4D-model) that considers in an exact manner the evolving of three-dimensional patient MR data over time.

In accordance with an embodiment of the invention, the scanning system is a magnetic resonance imaging system. However, as discussed above, other types of scanning systems, like for example CT scanning systems may be used.

In accordance with an embodiment of the invention, the simulating comprises instructing the patient to perform the physical or mental activity, wherein the evaluating of the recorded physiological data comprises analyzing the reaction time of the patient for performing the physical activity in response to the instructing, the chosen imaging scan protocol being a functional magnetic resonance, fMRT, imaging scan protocol for determining a neuronal activity of the patient associated with the physical or mental activity, the planning of the imaging scan protocol based on the chosen imaging scan protocol comprising synchronizing the fMRT imaging scan protocol with the analyzed reaction time of the patient.

In case for example elderly patients have a rather long reaction time for carrying out a certain instruction, the data acquisition for acquiring the fMRT data may be delayed in a certain manner without losing any significant information. However, the data acquisition should not be delayed for too long time periods, But when a correct synchronization between task/instruction and reaction is ensured, noise and wrong correlations in the data may be significantly reduced or be traded for imaging time.

In another aspect, the invention relates to a computer program product comprising computer executable instructions for performing the above described method.

In another aspect, the invention relates to a system for planning an imaging scan protocol to be performed by a scanning imaging system, the system comprising a virtual reality system, a memory containing machine executable instructions and a processor, wherein execution of the machine executable instructions causes the processor to: control the virtual reality system for simulating for a patient an imaging scan, the imaging scan following a chosen imaging scan protocol; control the system for recording physiological data of the patient during the simulating; control the system for modifying the chosen imaging scan protocol based on the physiological data.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image is defined herein as being the reconstructed two or three dimensional visualization of anatomic data contained within the magnetic resonance imaging data.

### BRIEF DESCRIPTION OF THE DRAWINGS

In the following preferred embodiments of the invention will be described, by way of example only, and with reference to the drawings in which:
Fig. 1 illustrates and example of a magnetic resonance imaging system,
Fig. 2 depicts a virtual reality headset together with a computing system,
Fig. 3 is a schematic of a patient subject to a virtual reality simulation using a virtual reality headset,
Fig. 4 is a flowchart of a method for planning an imaging scan protocol,

### DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

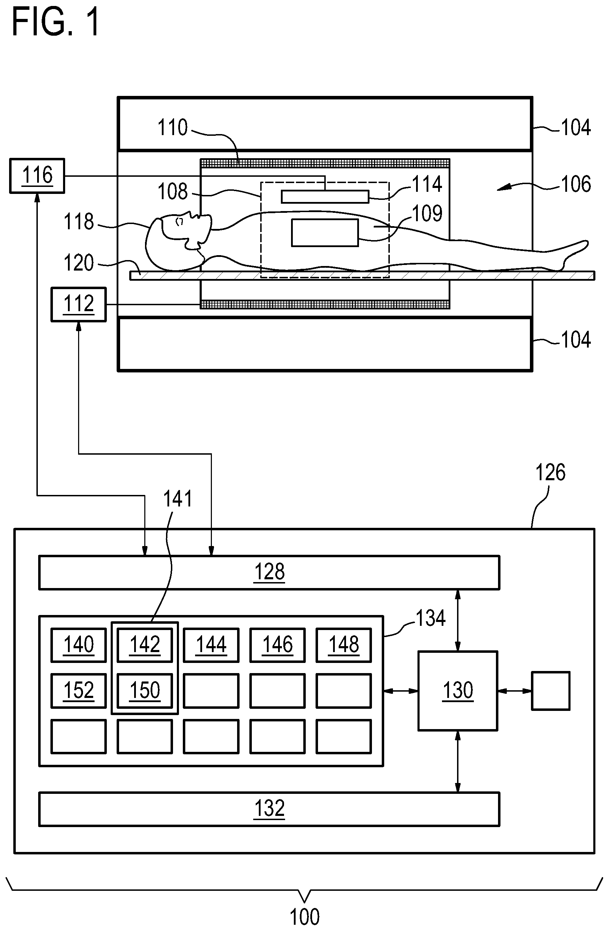

Fig. 1 shows an example of a magnetic resonance imaging system 100 with a magnet 104. As already mentioned, the above described principles of the invention can be applied to any imaging system like computer tomography, projectional radiography, diagnostic sonography etc. Therefore, the subsequent discussion regarding MRI shall not be considered as being limiting to MRI.

The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging. A region of interest 109 is shown within the imaging zone 108. A subject 118, for example a patient, is shown as being supported by a subject support 120, for example a moveable table, such that at least a portion of the subject 118 is within the imaging zone 108 and the region of interest 109.

Within the bore 106 of the magnet there is also a set of magnetic field gradient coils 110 which is used for acquisition of magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

The transceiver 116 and the gradient controller 112 are shown as being connected to a hardware interface 128 of a computer system 126. The computer system further comprises a processor 130 that is in communication with the hardware system 128, a memory 134, and a user interface 132. The memory 134 may be any combination of memory which is accessible to the processor 130. This may include such things as main memory, cached memory, and also non-volatile memory such as flash RAM, hard drives, or other storage devices. In some examples the memory 130 maybe considered to be a non-transitory computer-readable medium.

The computer memory 134 is shown as containing machine-executable instructions 140. The machine-executable instructions contain commands or instructions which enable the processor 130 to control the operation and function of the magnetic resonance imaging system 100. The computer memory 134 is shown as further containing imaging scan protocols 141. Each imaging scan protocol may comprise pulse sequence commands 142 for one or multiple pulse sequences which are either instructions or data which maybe converted into instructions which enable the processor 130 to control the magnetic resonance imaging system 100 to acquire magnetic resonance data. The pulse sequence commands 142 may therefore be part of an imaging scan protocol. The magnetic resonance data may for instance be used to cause the magnetic resonance imaging system to perform multiple pulse repetitions which cause magnetic resonance signals 144 to be acquired.

Magnetic resonance signals 144 are shown as being stored in the computer memory 134. The magnetic resonance signals 144 for a particular pulse repetition maybe collated into the magnetic resonance data 146. The magnetic resonance data 146 may be used to generate a series of images 148. The imaging scan protocols may further comprise instructions 150 regarding the reconstruction of the image data 146 acquired using the imaging.

Optionally, the memory 134 further comprises either raw or pre-processed physiological data 152 of the patient 118. The physiological data was previously recorded during a simulation of an imaging scan using a virtual reality system. This will be described in more details below.

The purpose of the physiological data 152 stored in the memory 134 is for example to synchronize the acquisition of MR images with the certain breathing pattern of the patient 118 which pattern can be extracted from the physiological data 152. As a result, MR image data can always be acquired in such a manner that with a rather high probability the image data is acquired at the same breathing phase of the patient. In another example, it is possible to use the physiological data 152 in combination with the instructions 150 for performing a highly accurate reconstruction of the image data 146. Again referring to the example of a breathing pattern extracted from the physiological data 152, the pattern can be used during the reconstruction for performing motion compensation on the image data 146. By means of the pattern, a more robust tracking of anatomical features recorded with the image data 146 may be possible.

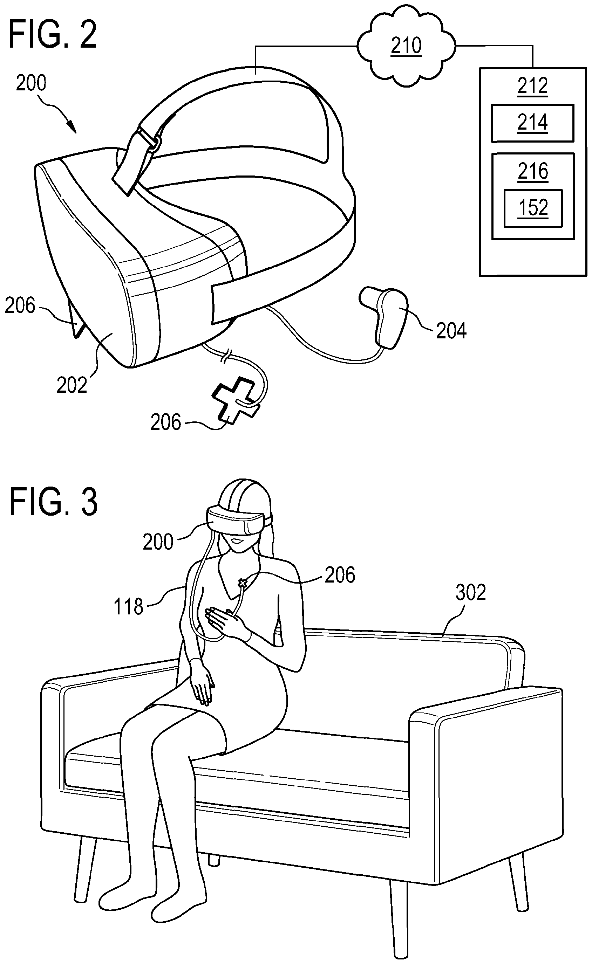

Fig. 2 depicts a virtual reality system which comprises a headset 200. The headset has the built-in screen 202 and speakers 204. In case a person is wearing the headset, the person is immersed in a three dimensional environment which can be visually experienced using the screen 202 and audibly be experienced using the speakers 204. Sensors 206 are further provided in order to recorded physiological data of the person.

As schematically depicted in Fig. 3, a person 118 may be sitting on a chair or a sofa 302 either at home or in the waiting room of a radiology office. The person 118 may be scheduled for being subject to a magnetic resonance imaging scan using the magnetic resonance scanner that was discussed above with respect to Fig. 1. For example, a chosen imaging scan protocol was preselected for the patient 118 by a medical doctor or radiologist.

A three-dimensional model of the magnetic resonance scanner including the bore 106 and the patient support 120 is simulated to the patient using the screen 202. Referring to the situation depicted in Fig. 1, it may be simulated in a three-dimensional manner to the patient 118 how he/she is currently located within the bore 106. Any noises made by the patient support 120 during the movement of the patient into the bore 106 may be simulated using the speakers 204. Further, performing an imaging scan following the chosen imaging scan protocol may also be simulated to the patient by mimicking the acoustic impressions on the patient that he/she would have when being subject to the imaging scan in reality. For example, the performing of the imaging scan may result in noises generated due to the operation of the gradient coils 110. Further, acoustic commands given by the radiologist to the patient may be simulated as will be explained in more detail below.

During the simulation of the imaging scan, physiological data of the patient 118 is acquired using the sensors 206. For example, the sensors 206 may acquire an actual heart rate, an actual blood pressure, skin conductance, pupil movement, neuronal activity (EEG), breathing pattern etc. of the patient. The acquired physiological data may then be stored in a respective computer system 212. The computer system and 212 may comprise a processor 214 and a memory 216, wherein the memory 216 is storing the physiological data 152. The memory 216 that may correspond to the memory 134 and the processor 214 may correspond to the processor 130 of Fig. 1. Thus, the computer system 212 may correspond to the to the computer system 126.

However, it is also possible that the computing system 212 is operable independent of the magnetic resonance imaging system of Fig. 1 and specially designed to control the headset 200, store the physiological data 152 and optionally analyze the physiological data 152. All this can be done either by direct wired connection between the headset 200 and the computing system 212 or for example via a network 210, the network being an intranet or the Internet.

In a practical example, the patient 118 depicted in Fig. 3 sitting on the sofa 302 is realistically experiencing the situation when lying on the patient support 120, being moved into the bore 106 and then being subject to an imaging scan. By for example monitoring the blood pressure of the patient and the skin conductivity of the patient versus time, the stress level of the patient maybe determined. For example, the computing system 212 may combine the blood pressure and the skin conductivity to obtain an analytical number representing the stress level of the patient. Further, the computing system 212 may comprise a predefined threshold level of the maximum stress level the patient should be subject to during the imaging scan. In case the computing system 212 determines that under the current conditions of simulating the imaging scan the patient's stress level is above the predefined threshold level, either the computing system 212 may modify the chosen imaging scan protocol or the computing system 212 may send electronic instructions to the magnetic resonance imaging system the 100 of Fig. 1 for modifying the chosen imaging scan protocol to be applied when the patient is subject to the imaging in reality.

Modifying the chosen imaging scan protocol may thereby performed in such a manner that it is ensured that when performing the imaging scan protocol in reality the stress level of the patient will be at or below the threshold level. For example, the patient may feel an anxiety state due to a claustrophobia when being located within the bore 106. This anxiety state is reflected and detected via a certain sweating and heart rate of the patient. Further, the computing system 212 may analyze that the patient is not sensitive regarding his anxiety state to noise emissions resulting from performing the imaging scan. Therefore, a modification of the chosen imaging protocol may comprise increasing the operation levels and thus operation currents of the gradient coils to the maximum allowed level. Even though this may significantly increase the noise emissions during the real imaging scan, the higher magnetic fields of the gradient coils may permit to perform certain imaging scans in a faster manner without the loss of image quality. Thus, the total time the patient has to stay in the bore 106 is reduced.

Further, in this example modifying the chosen imaging protocol may comprise the re-arrangement of individual parts sequences comprised in the protocol in such a manner that most relevant pulse sequences are performed first, followed by the less important parts sequences. For that, the individual parts sequences in the chosen imaging protocol may be ranked according to the priority. Thus, optional pulse sequences may be moved to the end of the imaging protocol such that in case of the necessity of aborting the imaging scan due to acute anxiety states of the patient the most important image data is already acquired and it is avoided that the patient has to be subject to the imaging for a second time only for the purpose of completing rather relevant acquired image data.

As mentioned above, acoustic instructions by the radiologist may also be simulated using the speakers 204. In an example, it is the goal to perform a breath holding technique in which MRI data can be acquired from the patient without movement of the patient's belly, as well as some of the patient's organs that may be subject to movement due to the breathing. The radiologist may give a spoken command to the patient asking him to hold breath. By means of the physiological sensors 206, the reaction capabilities of the patient can be monitored during the simulation and within certain limits be exercised and trained. In case the patient's reaction capabilities are reduced there maybe a rather long time span between giving the acoustic command to the patient and the patient understanding and comprehending. In turn, the chosen imaging protocol may be modified in such a manner that MR data acquisition is started only after a certain time delay after giving the command. During the real imaging scan, giving the command may be automated, computer controlled and computer executed using instructions of the imaging protocol.

Further, the physiological sensors 206 can monitor the breathing behavior of the patient. From the physiological data 152 regarding the patient's breathing activities in response to the command, the computing system 212 may calculate specifically for the actual patient a minimum guaranteed and steady breath hold phase. For example, the result may be "breath hold command, 5sec reaction time, 2sec taking deep breath, 15sec holding breath, 40sec recovery phase for next breath hold command". In turn, the chosen imaging protocol may be modified in such a manner that any MR measurements to be performed are only performed in the guaranteed 15 seconds breath hold phase. This minimizes the waste of data that is unusable due to breathing induced motion artifacts.

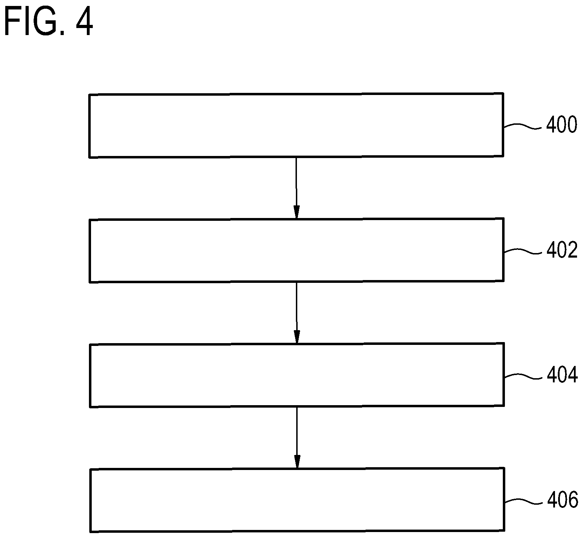

Fig. 4 is a flowchart illustrating a method for planning an imaging scan protocol to be performed by a scanning imaging system, like the scanning imaging system 100 of Fig. 1. The method starts in block 400 with the simulation the by the virtual reality system of an imaging scan to the patient. Here the imaging scan is following a chosen imaging scan protocol. In block 402, during the simulation the physiological data of the patient is recorded. The physiological data may be analyzed and accordingly the chosen imaging scan protocol is modified based on the physiological data in block 404. Finally, in block 406 the imaging scan using the modified chosen imaging scan protocol is performed on the patient.

In summary, many patients are unfamiliar with the setting, workflow and specific situation of medical (e.g. imaging) procedures resulting in anxiety behavior. This leads to workflow interruptions, unnecessary repetitions (e.g. image retakes), reduced (image) quality and suboptimal diagnostic/therapeutic outcome. The proposed method and device employs virtual reality to put the patient in a realistic setting, allowing him/her to go through the workflow at his/her own pace, retrieve additional information and thereby get accustomed to the upcoming procedure. Furthermore the device will detect/examine parameters which are relevant for the medical procedure, e.g. capability/willingness for breath-holds, presence of claustrophobia. If needed certain actions required to be performed by the patient could also be exercised/trained.

The virtual reality device can be any suitable, i.e. reliable, durable, ergonomic, hardware. The physiology information can be retrieved via e.g. a wrist band or camera-based system. For example, an imaging device-dependent virtual reality model including visualization and sound, as well as optionally an imaging site-specific virtual reality (VR) layout may be simulated to the patient. This includes realistic interactive workflow simulation that may even be patient/procedure-specific. Trainings and exercises (e.g. holding breath) may be performed using the VR simulation. From recorded physiological data of the patient, patient specific parameters (e.g. anxiety) may be derived and the parameters may then be used for modifying the chosen and simulated imaging scan protocol based on the physiological data.

### LIST OF REFERENCE NUMERALS

- 100: magnetic resonance imaging system
- 104: magnet
- 106: bore of magnet
- 108: imaging zone
- 109: region of interest
- 110: magnetic field gradient coils
- 112: magnetic field gradient coil power supply
- 114: radio-frequency coil
- 116: transceiver
- 118: subject
- 120: subject support
- 126: computer system
- 128: hardware interface
- 130: processor
- 132: user interface
- 134: computer memory
- 140: machine executable instructions
- 141: imaging scan protocol
- 142: pulse sequence commands
- 144: magnetic resonance signals
- 146: magnetic resonance data
- 148: intermediate images
- 150: image reconstruction instructions
- 152: physiological data
- 200: VR headset
- 202: screen
- 204: speaker
- 206: sensor
- 210: network
- 212: computing system
- 214: processor
- 216: memory
- 302: couch
- 400: simulating for a patient by a virtual reality system an imaging scan, the imaging scan following a chosen imaging scan protocol
- 402: recording physiological data of the patient during the simulating
- 404: modifying the chosen imaging scan protocol based on the physiological data
- 406: performing the imaging scan using the modified chosen imaging scan protocol

## Claims

1. A method for planning an imaging scan protocol to be performed by a scanning imaging system (100), the method comprising:
- simulating for a patient (118) by a virtual reality system (200) an imaging scan, the imaging scan following a chosen imaging scan protocol (141),
- recording physiological data (152) of the patient (118) during the simulating,
- modifying the chosen imaging scan protocol (141) based on the physiological data (152).

2. The method of claim 1, the virtual reality system (200) comprising sensors (206), the recording of the physiological data (152) being performed using the sensors (206).

3. The method of claim 2, the virtual reality system (200) comprising a virtual reality headset for performing the simulation, the sensors (206) being comprised in the headset.

4. The method of any of the previous claims, further comprising generating a 3D model of the scanner to be used for performing the imaging scan and generating an acoustic model of the noise emissions of the scanner resulting from performing the imaging scan, the simulating comprising an optical visualization of the imaging scan using the 3D model and an acoustic simulation of the imaging scan using the acoustic model, the optical visualization and the acoustic simulation mimicking the visual and acoustic impressions on the patient (118) when the patient (118) is subject to the imaging scan in reality.

5. The method of any of the previous claims, the physiological data (152) comprising anyone of the following:
- blood pressure,
- skin conductance,
- heart rate,
- breath rate,
- breathing pattern,
- blood oxygen saturation,
- pupil width,
- pupil movement,
- muscle tension (EMG)
- neuronal activity (EEG)
- skin temperature,
- reaction time for performing a physical or mental activity,
- breath hold duration.

6. The method of any of the previous claims, the method further comprising anyone of
- in case a combination of values of the physiological data (152) is above a predefined threshold level, modifying the chosen imaging scan protocol (141) such that the combination of values of the physiological data (152) to be expected when performing the imaging scan protocol in reality is reduced below the threshold level,
- in case the combination of values of the physiological data (152) is below a predefined threshold level, modifying the chosen imaging scan protocol (141) such that the combination of values of the physiological data (152) level to be expected when performing the imaging scan protocol in reality is at the threshold level.

7. The method of claim 6, the modification of the chosen imaging scan protocol (141) comprising anyone of: a modification of the time duration of the chosen imaging scan protocol (141), a modification of the noise level resulting from performing the chosen imaging scan protocol (141), a modification of the image acquisition parameters of the chosen imaging scan protocol (141), a modification of the image reconstruction parameters of the chosen imaging scan protocol (141), a splitting of the chosen imaging scan protocol (141) into a set of scan sequences (142) separated in time from each other, rearranging the order of a set of scan sequences (142) comprised in the chosen imaging scan protocol (141).

8. The method of claim 5, further comprising analyzing the breathing pattern for identifying the time and/or amplitude behavior of reoccurring breathing induced movement states of the patient's (118) body, wherein the modifying of the chosen imaging scan protocol (141) is performed such that image data acquisition is adapted to and/or synchronized with the time behavior.

9. The method of claim 5, further comprising performing the imaging scan using the imaging scan protocol, the performing of the imaging scan resulting in image data (146), the method further comprising performing a motion compensation of the image data (146) using the breathing pattern.

10. The method of any of the previous claims, the scanning imaging system (100) being a magnetic resonance imaging system.

11. The method of claim 10, the simulating comprising instructing the patient (118) to perform the physical or mental activity, wherein the evaluating of the recorded physiological data (152) comprises analyzing the reaction time of the patient (118) for performing the physical activity in response to the instructing, the chosen imaging scan protocol (141) being a functional magnetic resonance, fMRT, imaging scan protocol for determining a neuronal activity of the patient (118) associated with the physical or mental activity, the planning of the imaging scan protocol based on the chosen imaging scan protocol (141) comprising synchronizing the fMRT imaging scan protocol with the analyzed reaction time of the patient (118).

12. A computer program product comprising computer executable instructions for performing the method as claimed in any of the previous claims.

13. A system for planning an imaging scan protocol to be performed by a scanning imaging system (100), the system comprising a virtual reality system (200), a memory containing machine executable instructions and a processor, wherein execution of the machine executable instructions causes the processor to:
- control the virtual reality system (200) for simulating for a patient (118) an imaging scan, the imaging scan following a chosen imaging scan protocol (141),
- control the system for recording physiological data (152) of the patient (118) during the simulating,
- control the system for modifying the chosen imaging scan protocol (141) based on the physiological data (152).
